# FASCICULE DE BREVET EUROPEEN

(11) **EP 4 013 433 B1**
(45) Date de publication et mention de la délivrance du brevet: **20.11.2024**
(21) Numéro de dépôt: 20761761.4
(22) Date de dépôt: 12.08.2020
(51) Int. Cl.: A61K 35/30, A61F 2/14, A61L 27/00, A61L 27/36, A61P 27/02

(54) **UNITE TRIDIMENSIONNELLE CREUSE DE TISSU RETINIEN ET UTILISATION DANS LE TRAITEMENT DES RETINOPATHIES**
HOHLE DREIDIMENSIONALE EINHEIT AUS RETINALEM GEWEBE UND DEREN VERWENDUNG ZUR BEHANDLUNG VON RETINOPATHIEN
HOLLOW THREE-DIMENSIONAL UNIT MADE FROM RETINAL TISSUE AND USE THEREOF IN THE TREATMENT OF RETINOPATHIES

(30) Priorité: 12.08.2019 FR 1909155
(43) Date de publication de la demande: 22.06.2022
(73) Titulaire: Treefrog Therapeutics, 33600 Pessac (FR)
(72) Inventeur: FEYEUX, Maxime, 33400 TALENCE (FR); ALESSANDRI, Kevin, 33000 BORDEAUX (FR)
(74) Mandataire: Aquinov
(86) Numéro de dépôt international: PCT/EP2020/072567
(87) Numéro de publication internationale: WO 2021/028456

(56) Documents cités:
- WO-A1-2017/176810
- WO-A1-2018/096277
- FR-A1- 3 058 892
- FR-A1- 3 059 009
- KEVIN ALESSANDRI ET AL: "A 3D printed microfluidic device for production of functionalized hydrogel microcapsules for culture and differentiation of human Neuronal Stem Cells (hNSC)", LAB ON A CHIP, vol. 16, no. 9, 1 January 2016 (2016-01-01), pages 1593 - 1604, XP055393107, ISSN: 1473-0197, DOI: 10.1039/C6LC00133E
- HUNT NICOLA C ET AL: "3D culture of human pluripotent stem cells in RGD-alginate hydrogel improves retinal tissue development", ACTA BIOMATERIALIA, ELSEVIER, AMSTERDAM, NL, vol. 49, 5 November 2016 (2016-11-05), pages 329 - 343, XP029885863, ISSN: 1742-7061, DOI: 10.1016/J.ACTBIO.2016.11.016
- FRANCE 3 NOUVELLE-AQUITAINE: "Portrait de la start-up TreeFrog, à Bordeaux, spécialisée dans la production de cellules souches", YOUTUBE, 19 March 2019 (2019-03-19), pages 1 pp., XP054980450, Retrieved from the Internet <URL:https://www.youtube.com/watch?v=gUvQFLJkaCs> [retrieved on 20200507]
- BRYCE T. MCLELLAND ET AL: "Transplanted hESC-Derived Retina Organoid Sheets Differentiate, Integrate, and Improve Visual Function in Retinal Degenerate Rats", INVESTIGATIVE OPTHALMOLOGY & VISUAL SCIENCE, vol. 59, no. 6, 22 May 2018 (2018-05-22), pages 2586, XP055598803, ISSN: 1552-5783, DOI: 10.1167/iovs.17-23646

## Description

### Domaine technique

La présente invention concerne le traitement des maladies de la rétine, en particulier par l'utilisation d'unités de tissu spécifiques comprenant au moins un épithélium pigmentaire rétinien. L'invention concerne également un procédé de préparation de ces unités de tissu ainsi que des kits d'implantation dans l'oeil de ces unités de tissu pour réaliser des greffes de tout ou partie de la rétine.

### Art antérieur

Les maladies de la rétine, ou rétinopathies, constituent une des causes majeures de malvoyance dans le monde. La rétine, qui tapisse le fond de l'oeil, est constituée notamment de cellules épithéliales pigmentaires et de cellules nerveuses qui reçoivent la lumière. Ces cellules nerveuses traduisent la lumière en signaux électriques qui transitent vers le cerveau via le nerf optique. Lorsque les cellules de la rétine, en particulier de l'épithélium pigmentaire rétinien, dégénèrent ou ne fonctionnent plus, des zones aveugles du champ visuel apparaissent.

Les rétinopathies peuvent avoir des origines variées : elles peuvent notamment être liées au vieillissement, comme la dégénérescence maculaire liée à l'âge (DMLA), être héréditaires, comme la rétinopathie pigmentaire ou dystrophie rétinienne, être liées à un traumatisme comme la rétinopathie solaire, ou dériver d'une autre pathologie, comme la rétinopathie diabétique ou la rétinopathie hypertensive.

La DMLA représente la première cause de malvoyance chez la personne âgée. Cette pathologie est due à l'atteinte de la macula, zone centrale de la rétine, qui transmet l'essentiel de l'information visuelle au cerveau. Elle se traduit par l'apparition d'une tâche aveugle au centre du champ visuel. La DMLA peut se présenter sous deux formes :
- une forme sèche ou atrophique : elle est caractérisée par la disparition progressive des cellules de la macula. Elle évolue donc lentement et constitue la forme la plus fréquente de DMLA;
- une forme humide ou exsudative : elle est caractérisée par la formation de vaisseaux sanguins anormaux sous la rétine qui conduisent notamment à son décollement.

Il n'existe actuellement aucun traitement contre la forme sèche de DMLA. Le seul moyen utilisé est de la retarder en prenant des compléments alimentaires (vitamines C, E et minéraux antioxydants). Depuis quelques années, pour la DMLA humide, si la maladie n'est pas à un stade trop avancé, une injection dans l'oeil d'un médicament qui bloque la prolifération des vaisseaux peut permettre une amélioration de la maladie mais ce traitement nécessite une injection mensuelle et ne permet pas d'obtenir des résultats entièrement satisfaisants.

La rétinopathie pigmentaire est une maladie génétique qui peut être d'origine héréditaire. Elle est caractérisée par une dégénérescence des cellules de la rétine liée à la mutation d'un ou plusieurs gènes. L'évolution de la maladie est lente jusqu'à entraîner une cécité et il n'existe pour l'instant pas de traitement.

La rétinopathie diabétique est une atteinte rétinienne survenant dans le cadre d'un diabète. Elle est liée à la concentration en sucre excessive au niveau des petits vaisseaux sanguins de la rétine, ce qui conduit à leur dégradation. Le manque d'apport en oxygène induit la formation de nouveaux vaisseaux sanguins, plus fragiles. Leur rupture et les microhémorragies qui s'ensuivent peuvent conduire à un décollement de la rétine. Comme pour la forme humide de la DMLA il est possible de réaliser des injections de médicaments anti-angiogéniques mais ce traitement fonctionne mal. Un traitement laser peut également être réalisé pour brûler les vaisseaux anormaux mais l'efficacité est limitée.

Récemment de nouvelles approches thérapeutiques ont été décrites dans un objectif de traitement des maladies de la rétine.

De récents essais thérapeutiques ont exploré la possibilité de poser un implant rétinien (rétine artificielle) mais la résolution de cet implant reste faible.

Des essais en thérapie cellulaire ont également été menés dans le but de remplacer les cellules de la rétine qui ont dégénéré par des cellules souches capables de se différencier en cellules épithéliales, neurales ou vasculaires de la rétine (hRPCs ou « human retinal progenitor cells » cellules progénitrices rétinienne humaine). Toutefois aucun essai n'a été concluant à ce jour, en particulier la différenciation cellulaire après implantation n'est pas contrôlée et l'injection intravitréale qui est la voie d'administration testée ne correspond pas à un mécanisme physiologique, ce qui a conduit à des effets secondaires indésirables (https://clinicaltrials.gov/ct2/show/results/NCT02320812). Par ailleurs ces cellules progénitrices existent in vivo chez l'homme sans qu'elles ne permettent une régénération de la rétine adulte (Tang et al. « Progress of stem/progenitor cell-based therapy for retinal degeneration » Journal of Translational Medicine, 10 mai 2017).

Par ailleurs des membranes ou des feuilles de cellules rétiniennes pour leur utilisation comme implant ont été décrites. C'est le cas de la demande US20160310637 ou encore de la demande EP2570139 qui divulguent des membranes ou des feuilles de cellules de rétine obtenues à partir de cellules rétiniennes prélevées sur l'homme et cultivées, lesdites membranes ou feuilles étant destinées à être implantées dans l'oeil. Toutefois cette technologie n'est pas satisfaisante car elle nécessite de produire un nombre très important de cellules vis-à-vis du nombre de cellules greffées (8 lots produits pour permettre les contrôles qualité et la majorité des cellules dans chaque lot n'est pas positionnée sur l'implant), de plus, l'implantation demande de réaliser une chirurgie longue et complexe requérant une expertise de greffe spécifique pour le praticien et très invasive pour le patient.

La demande EP3211071 décrit également des tissus rétiniens sous forme d'agrégats en suspension obtenus à partir de cellules souches pluripotentes. La suspension est ensuite injectée dans l'oeil. Cette solution n'est pas non plus satisfaisante car l'injection ne permet pas de conserver une structuration fonctionnelle (notamment une polarisation fonctionnelle) : i) dans le cas de l'épithélium pigmentaire dont la face apicale des cellules doit être présentée en vis-à-vis des segments externes des photorécepteurs du greffon, limitant la survie et la fonctionnalité de la greffe ii) dans le cas des photorécepteurs le segment externe doit être positionné vers l'extérieur de l'oeil en vis-à-vis des cellules de l'épithélium pigmentaire et la terminaison synaptique faire face au centre de l'oeil pour connecter le reste de la rétine neurale). De plus, cette technique nécessite l'injection d'un volume important de liquide dans l'oeil, et conduit, comme avec toutes les autres solutions de l'art antérieur, à un décollement de la rétine important, sur une zone plus large que la zone à traiter au risque de provoquer des hémorragies locales lors de l'incision ou de l'injection. Les solutions actuelles nécessitent en outre la réalisation de trois ou quatre incisions comme décrit dans Zarbin et al. (« Concise Review: Update on Retinal Pigment Epithelium Transplantation for Age-Related Macular Degeneration » Stem Cells Translational Medicine, 2019;8:466-477). WO 2017/176810 décrit une unité de tissu rétinien tridimentionelle comprenant une structure laminaire concentrique comprenant un centre de cellules d'épithélium pigmentaire rétinien, une couche de RGCs, une couche de neurones rétiniens de second ordre, une couche de photorécepteurs et une couche de cellules d'épithélium pigmentaire rétinien ainsi qu'un hydrogel.

L'objectif de l'invention est de pallier ces différentes problématiques de l'art antérieur, et de proposer une solution pour l'implantation facile, rapide et peu invasive, de cellules de rétine correctement polarisées, et leur intégration dans l'organe hôte, de façon à remplacer de façon durable et certaine les cellules rétiniennes qui ont dégénéré chez des patients atteints de pathologies dégénératives de la rétine.

### Résumé de l'invention

Selon l'invention, l'efficacité de la greffe de tissus rétiniens dépend largement de la bonne incorporation in situ (notamment polarisation apico-basale correcte) du greffon via l'adhésion de la face basale des cellules de l'épithélium pigmentaire au niveau de la membrane de Bruch. C'est pourquoi, pour répondre à l'objectif de l'invention, les inventeurs ont mis au point des réseaux tridimensionnels cellulaires creux particuliers comprenant au moins une couche de cellules d'épithélium pigmentaire rétinien humaines vivantes différenciées, organisées autour d'une lumière, lesdites cellules de l'épithélium pigmentaire rétinien ayant leurs faces basales orientées vers l'extérieur. Cette unité de tissu comprend préférentiellement également une couche extérieure de matrice extracellulaire au niveau de la face basale des cellules d'épithélium pigmentaire rétinien favorisant l'intégration et la survie des cellules, une fois injectées dans l'oeil. L'unité de tissu selon l'invention peut également contenir d'autres cellules rétiniennes, organisées sous forme d'une ou plusieurs couches à l'intérieur de la couche de cellules d'épithélium pigmentaire rétinien, dans la lumière interne, c'est-à-dire au niveau de la face apicale des cellules d'épithélium pigmentaire rétinien. Ces autres cellules forment préférentiellement tout ou partie d'un tissu neural rétinien.

L'invention a donc pour objet une unité de tissu tridimensionnelle creuse, comprenant, organisée autour d'une lumière interne, au moins une couche de cellules d'épithélium pigmentaire rétinien humaines vivantes différenciées, dont la face basale de chaque cellule d'épithélium pigmentaire rétinien est positionnée vers l'extérieur et la face apicale vers la lumière interne. L'unité de tissu selon l'invention se présente préférentiellement sous la forme d'un ovoïde creux, d'un cylindre creux, d'un sphéroïde creux ou d'une sphère creuse, ou d'une section de ces éléments selon un plan.

L'invention a également pour objet une telle unité de tissu tridimensionnelle creuse pour son utilisation dans le traitement des maladies de la rétine, en particulier par implantation dans l'oeil, au niveau de la membrane de Bruch.

Avantageusement, la greffe étant réalisée avec des unités de tissu rétinien selon l'invention, organisées et de taille submillimétrique, l'intervention ne nécessite pas le positionnement précis d'un unique greffon comme dans l'art antérieur. Ceci limite le besoin de décollement (potentiellement traumatique) de la rétine et par conséquent également l'intensité du drainage de l'humeur vitrée de l'oeil. En outre, l'invention a l'avantage de pouvoir limiter l'intervention chirurgicale à une unique incision de l'oeil contre trois à quatre aujourd'hui.

De plus, la polarisation de la couche d'épithélium pigmentaire rétinien (face basale vers l'extérieur et la face apicale vers la lumière interne) permet aux unités de tissus rétiniens de se positionner correctement lorsqu'ils sont greffés dans l'oeil et d'assurer la réussite de la greffe. En effet grâce au positionnement de leur face basale coté extérieur, les unités de tissu rétinien selon l'invention s'attachent à la matrice extracellulaire du fond de l'oeil (membrane de Brusch) en émettant des cellules adhérentes au substrat qui migrent et forment une monocouche.

L'invention a aussi pour objet un procédé de préparation d'une unité de tissu rétinien tridimensionnelle creuse comprenant les étapes consistant à :
- produire un microcompartiment cellulaire comprenant, à l'intérieur d'une capsule en hydrogel :
   * des cellules de d'épithélium pigmentaire rétinien et éventuellement d'autres cellules rétiniennes, ou
   * des cellules aptes à se différencier en cellules d'épithélium pigmentaire rétinien et éventuellement en d'autres cellules rétiniennes,
   les cellules aptes à se différencier au moins en cellules d'épithélium pigmentaire rétinien étant choisies parmi :
   * des cellules souches capables de se différencier en cellules de la rétine, au moins en cellules d'épithélium pigmentaire rétinien,
   * des cellules progénitrices capables, de se différencier en cellules de la rétine, au moins en cellules d'épithélium pigmentaire rétinien,
   * des cellules différenciées capables de subir une trans-différenciation en cellules de la rétine, au moins en cellules d'épithélium pigmentaire rétinien,
   * des cellules différenciées capables de subir une reprogrammation de façon à ce qu'elles deviennent des cellules souches pluripotentes induites capables de se différencier en cellules de la rétine, au moins en cellules d'épithélium pigmentaire rétinien.
- si le microcompartiment comprend des cellules aptes à se différencier en cellules d'épithélium pigmentaire rétinien et éventuellement en d'autres cellules rétiniennes : induire la différenciation cellulaire au sein du microcompartiment cellulaire, de manière à obtenir des cellules d'épithélium pigmentaire rétinien et éventuellement d'autres cellules rétiniennes,
- éliminer les capsules d'hydrogel par hydrolyse, dissolution, perçage et/ou rupture pour récupérer les cellules d'épithélium pigmentaire rétinien et les éventuelles autres cellules rétiniennes sous forme d'une unité de tissu.

Enfin, l'invention a aussi pour objet un kit d'implantation dans l'oeil d'unités de tissu tridimensionnelles creuses comprenant au moins :
- entre 1 et 10 000 unités de tissu, éventuellement encapsulées dans des capsules d'hydrogel, et
- un dispositif d'implantation chirurgical apte à implanter la ou lesdites unité(s) de tissu dans un oeil humain.

### Brève description des Figures

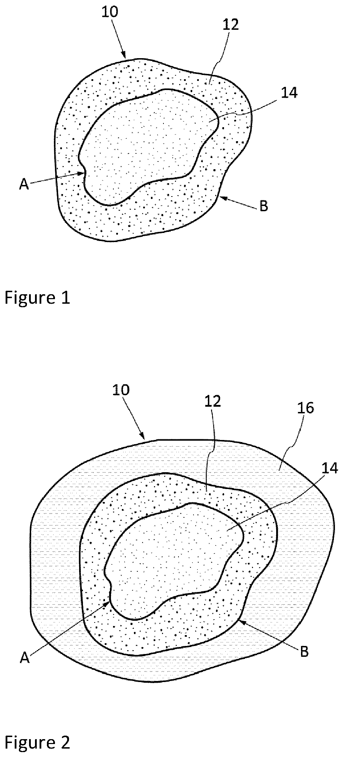

- Figure 1 : la figure 1 est une représentation schématique d'une vue en coupe d'une unité de tissu 10 selon l'invention, avec une couche d'épithélium pigmentaire rétinien 12 et une lumière interne 14, A correspondant à la face apicale des cellules d'épithélium pigmentaire rétinien et B correspondant à la face basale des cellules d'épithélium pigmentaire rétinien.
- Figure 2 : la figure 2 est une représentation schématique d'une vue en coupe d'une unité de tissu 10 selon l'invention, avec une couche d'épithélium pigmentaire rétinien 12, une lumière 14 et une couche de matrice extracellulaire 16, A correspondant à la face apicale des cellules d'épithélium pigmentaire rétinien et B correspondant à la face basale des cellules d'épithélium pigmentaire rétinien.

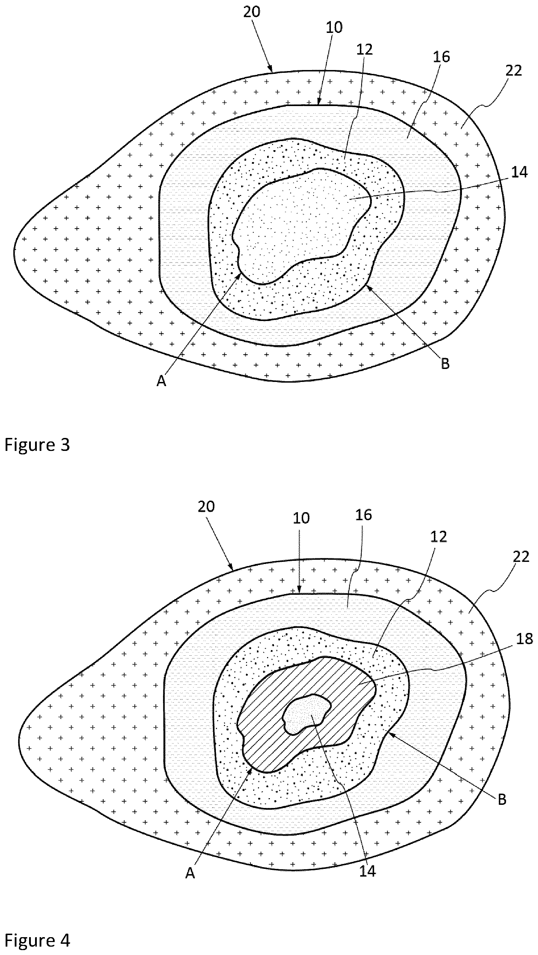

- Figure 3 : la figure 3 est une représentation schématique d'une vue en coupe d'un microcompartiment 20 comprenant une capsule d'hydrogel 22 et une unité de tissu 10 selon l'invention, l'unité de tissu 10 étant constituée par une couche d'épithélium pigmentaire 12, une lumière 14 et une couche de matrice extracellulaire 16, A correspondant à la face apicale des cellules d'épithélium pigmentaire rétinien et B correspondant à la face basale des cellules d'épithélium pigmentaire rétinien.
- Figure 4 : la figure 4 est une représentation schématique d'une vue en coupe d'un microcompartiment 20 comprenant une capsule d'hydrogel 22 et une unité de tissu 10 selon l'invention, l'unité de tissu 10 étant constituée par une couche de matrice extracellulaire 16, une couche d'épithélium pigmentaire rétinien 12, une couche de cellules rétiniennes autres que des cellules d'épithélium pigmentaire rétinien 18, et une lumière 14, A correspondant à la face apicale des cellules d'épithélium pigmentaire rétinien et B correspondant à la face basale des cellules d'épithélium pigmentaire rétinien.

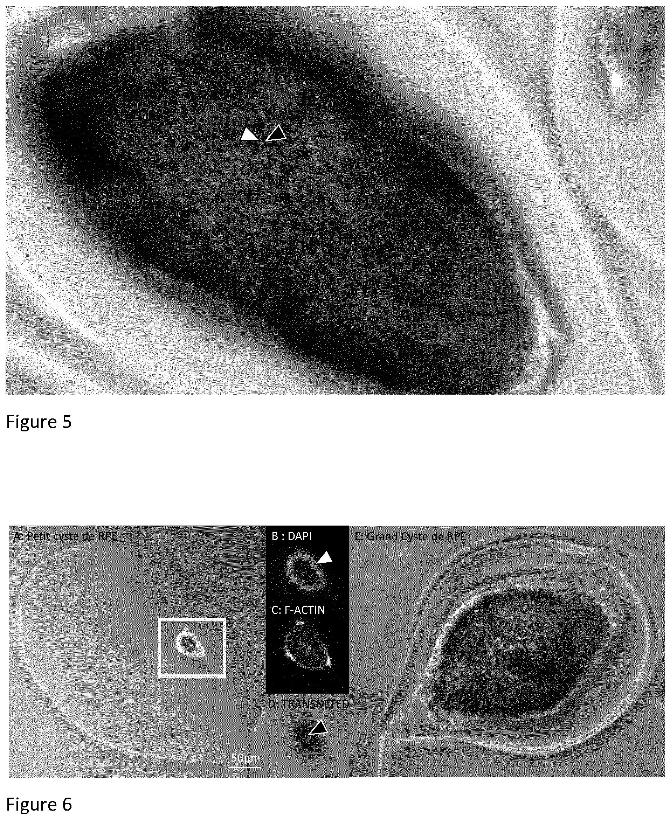

- Figure 5 : la figure 5 représente une image en microscopie d'un microcompartiment en hydrogel (alginate) encapsulant une unité de tissu selon l'invention.
- Figure 6 : la figure 6 représente des images en microscopie de microcompartiments en hydrogel (alginate) encapsulant une petite unité de tissu selon l'invention (A) et une grande unité de tissu selon l'invention (B).

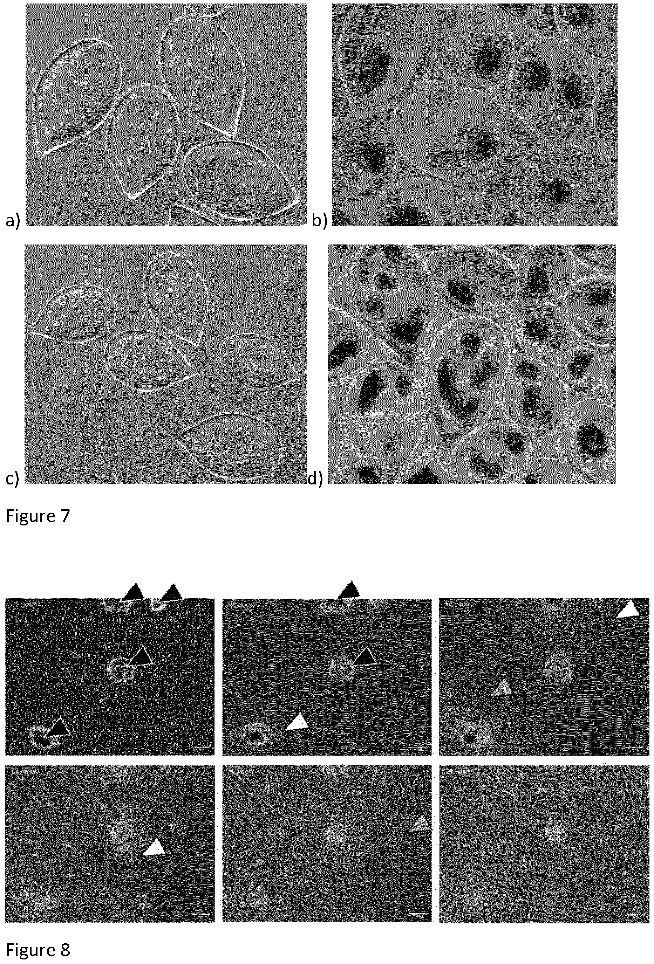

- Figure 7 : la figure 7 représente des images en microscopie de microcompartiments en hydrogel (alginate) encapsulant une ou plusieurs unités de tissu selon l'invention avec différentes densités cellulaires.
- Figure 8 : la figure 8 représente des unités de tissu rétinien selon l'invention, sans capsule d'hydrogel, sur un substrat simulant la matrice extracellulaire du fond de l'oeil (ici matrigel simulant la membrane de Brusch) à différents temps jusqu'à formation d'une monocouche d'épithélium pigmentaire rétinien.

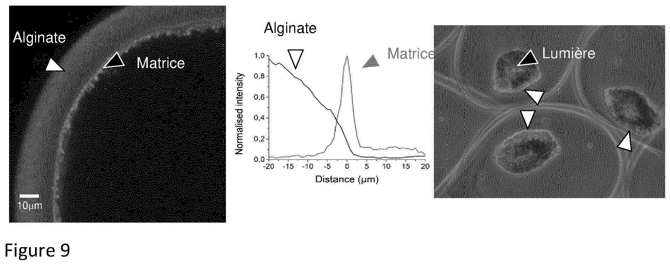

- Figure 9 : la figure 9 représente des images en microscopie et un graphe qui montrent que la polarisation des cellules peut être obtenue par déposition d'une couche de matrice sur la face interne de capsules d'alginate.

### Description détaillée de l'invention

### Définitions

Par « alginate » au sens de l'invention, on entend des polysaccharides linéaires formés à partir de β-D-mannuronate et α-L-guluronate, des sels et des dérivés de ceux-ci.

Par « capsule en hydrogel » au sens de l'invention, on entend une structure tridimensionnelle formée à partir d'une matrice de chaînes polymères, gonflée par un liquide et préférentiellement de l'eau.

Par « cellules humaines » au sens de l'invention on entend des cellules humaines ou des cellules de mammifères non humains immunologiquement humanisées. Même lorsque cela n'est pas précisé, les cellules, les cellules souches, les cellules progénitrices et les tissus selon l'invention sont constitués ou sont obtenus à partir de cellules humaines ou à partir de cellules de mammifères non humains immunologiquement humanisées.

Par « cellule progénitrice » au sens de l'invention, on entend une cellule souche déjà engagée dans la différenciation cellulaire en cellules de la rétine mais pas encore différenciée.

Par « cellule souche embryonnaire » au sens de l'invention on entend une cellule souche pluripotente de cellule dérivée de la masse cellulaire interne du blastocyste. La pluripotence des cellules souches embryonnaires peut être évaluée par la présence de marqueurs tels que les facteurs de transcription OCT4 et NANOG et des marqueurs de surface comme SSEA3/4, Tra-1-60 et Tra-1-81. Les cellules souches embryonnaires selon l'invention sont obtenues sans destruction de l'embryon dont elles sont issues, par exemple à l'aide de la technique décrite dans Chang et al. (Cell Stem Cell, 2008, 2(2)) : 113-117). Eventuellement les cellules souches embryonnaires d'êtres humains peuvent être exclues.

Par « cellule souche pluripotente » ou « cellule pluripotente » au sens de l'invention, on entend une cellule qui a la capacité de former tous les tissus présents dans l'organisme d'origine entier, sans pour autant pouvoir former un organisme entier en tant que tel. Il peut s'agir en particulier de cellules souches pluripotentes induites, de cellules souches embryonnaires ou de cellules MUSE (pour « Multilineage-differentiating Stress Enduring »). Par « cellule souche pluripotente induite » au sens de l'invention on entend une cellule souche pluripotente induite à la pluripotence par reprogrammation génétique de cellules somatiques différenciées. Ces cellules sont notamment positives pour les marqueurs de pluripotence, comme la coloration à la phosphatase alcaline et l'expression des protéines NANOG, SOX2, OCT4 et SSEA3/4. Des exemples de procédés permettant l'obtention de cellules souches pluripotentes induites sont décrits dans les articles Yu et al. (Science 2007, 318 (5858) : 1917-1920), Takahashi et al (Cell, 207, 131(5) : 861-872) et Nakagawa et al (Nat Biotechnol, 2008, 26(1) : 101-106).

Par cellules « différenciées » au sens de l'invention on entend des cellules qui présentent un phénotype particulier, par opposition à des cellules souches pluripotentes qui ne sont pas différenciées.

Par « diamètre de Feret » d'une unité de tissu on entend la distance « d » comprise entre deux tangentes à ladite unité de tissu, ces deux tangentes étant parallèles, de telle sorte que l'ensemble de la projection de l'unité de tissu soit comprise entre ces deux tangentes parallèles.

Par « implantation » ou « greffe » dans l'oeil au sens de l'invention, on entend l'action de déposer dans l'oeil à un endroit particulier, au moins une unité de tissu selon l'invention. L'implantation peut être réalisée par tout moyen notamment par injection.

Par « plus grande dimension » d'une unité de tissu au sens de l'invention, on entend la valeur du plus grand diamètre de Feret de ladite unité de tissu.

Par « plus petite dimension » d'une unité de tissu au sens de l'invention, on entend la valeur du plus petit diamètre de Feret de ladite unité de tissu.

Par « unité de tissu » ou « unité de tissu rétinien" selon l'invention, on entend une unité comprenant au moins un tissu de la rétine. L'unité de tissu rétinien peut comprendre plusieurs tissus de la rétine assemblés entre eux avec une structuration fonctionnelle. L'unité de tissu selon l'invention comprend au moins un tissu épithélial pigmentaire rétinien et il peut aussi contenir un autre tissu rétinien notamment un tissu neural rétinien ou vasculaire rétinien et/ou au moins un autre constituant comme par exemple une matrice extracellulaire.

### Unité de tissu

L'invention a donc pour objet une unité de tissu rétinien tridimensionnelle.

L'unité de tissu selon l'invention est creuse. Elle comprend toujours une lumière interne ou lumen qui constitue la partie creuse de l'unité de tissu. Cette lumière est générée au moment de la formation de l'unité de tissu par les cellules rétiniennes qui se multiplient et se développent. La lumière contient un liquide, notamment un milieu de culture (tel qu'un milieu basé sur le DMEM ou le DMEM-F12 et/ou le Neurobasal et complémenté avec du B27 ou N-2 ou NS21) et/ou un liquide sécrété par les cellules de l'unité de tissu. Avantageusement la présence de cette partie creuse dans l'unité de tissu rétinien permet une meilleure intégration au niveau de la rétine lorsqu'elle est implantée dans l'oeil.

L'unité de tissu selon l'invention comprend au moins une couche de cellules d'épithélium pigmentaire rétinien. Ces cellules sont des cellules humaines, vivantes et différenciées en cellules d'épithélium pigmentaire rétinien. La couche de cellules d'épithélium rétinien est organisée autour de la lumière interne. Les cellules constituant cette couche forment ensemble un épithélium pigmentaire rétinien, et leurs faces basales sont toutes positionnées en direction de l'extérieur de l'unité de cellules, et leurs faces apicales sont toutes positionnées en direction de l'intérieur c'est-à-dire vers la lumière interne. Préférentiellement, les cellules juxtaposées sont liées entre elles sur leurs faces latérales par des jonctions serrées.

Selon un mode de réalisation particulièrement adapté, l'unité de tissu selon l'invention comprend également une couche externe de matrice extracellulaire. Cette couche externe de matrice extracellulaire est située au niveau de la face basale des cellules d'épithélium pigmentaire rétinien. La couche de matrice cellulaire peut être constituée par de la matrice cellulaire sécrétée par des cellules d'épithélium pigmentaire rétinien et/ou par de la matrice extracellulaire ajoutée au moment de la préparation de l'unité de cellules.

La couche de matrice extracellulaire peut former un gel. Elle comprend un mélange de protéines et de composés extracellulaires nécessaires à la culture des cellules d'épithélium rétinien pigmentaire. Préférentiellement, la matrice extracellulaire comprend des protéines structurelles, telles que du collagène, des laminines, de l'entactine, de la vitronectine, ainsi que des facteurs de croissance, tels que du TGF-béta et/ou de l'EGF. La couche de matrice extracellulaire peut consister en ou comprendre du Matrigel^{®} et/ou de la Geltrex^{®} et/ou une matrice type hydrogel d'origine végétale comme des alginates modifiés ou d'origine synthétique ou de copolymère de poly(N-isopropylacrylamide) et de poly(ethylene glycol) (PNIPAAm-PEG) type Mebiol^{®}.

Au niveau de la surface de la couche de matrice extracellulaire en contact avec la couche d'épithélium pigmentaire rétinien, la matrice extracellulaire peut éventuellement contenir une ou plusieurs cellules d'épithélium pigmentaire rétinien.

Lorsque la matrice extracellulaire est présente, avantageusement les cellules rétiniennes organisées en trois dimensions autour de la lumière interne, interagissent déjà avec une matrice extracellulaire, ce qui facilite leur implantation au niveau de la rétine.

L'unité de tissu selon l'invention peut comprendre une ou plusieurs autres couches de cellules rétiniennes autres que des cellules d'épithélium pigmentaire rétinien. Ces cellules sont des cellules humaines, qui sont vivantes et différenciées en cellules rétiniennes autres que des cellules d'épithélium pigmentaire rétinien. La ou les couches de cellules rétiniennes autres que des cellules d'épithélium pigmentaire rétinien sont disposées à l'intérieur de la couche de cellules d'épithélium rétinien, c'est-à-dire au niveau de la face apicale des cellules d'épithélium pigmentaire rétinien, organisée(s) autour du lumen.

Dans un mode de réalisation, les cellules rétiniennes autres que des cellules d'épithélium pigmentaire rétinien sont choisies parmi les bâtonnets, les cônes, les cellules ganglionnaires, les cellules amacrines, les cellules bipolaires et les cellules horizontales.

Lorsque l'unité cellulaire comprend au moins deux couches de cellules rétiniennes autres que des cellules d'épithélium pigmentaire rétinien, les différentes couches sont organisées successivement autour de la lumière interne.

Préférentiellement, l'unité de tissu selon l'invention contient entre 10 et 100 000 cellules rétiniennes.

Selon un mode de réalisation particulier de l'invention, tel que représenté sur la figure 1, l'unité de tissu rétinien tridimensionnelle creuse 10 est constituée exclusivement :
- d'une lumière interne 14, et
- d'une couche d'épithélium pigmentaire rétinien 12 organisée autour de la lumière interne, les faces basales B des cellules épithéliales pigmentaires rétiniennes étant dirigées vers l'extérieur de l'unité de tissu, et les faces apicales A des cellules épithéliales pigmentaires rétiniennes étant dirigées vers la lumière interne.

Selon un autre mode de réalisation particulier de l'invention, tel que représenté sur la figure 2, l'unité de tissu rétinien tridimensionnelle creuse 10 est constituée exclusivement :
- d'une lumière interne 14,
- d'une couche d'épithélium pigmentaire rétinien 12 organisée autour de la lumière interne, les faces basales B des cellules épithéliales pigmentaires rétiniennes étant dirigées vers l'extérieur de l'unité de tissu, et les faces apicales A des cellules épithéliales pigmentaires rétiniennes étant dirigées vers la lumière interne, et
- d'une couche de matrice extracellulaire 16 disposée autour de la couche de cellules d'épithélium pigmentaire rétinien, au niveau des faces basales desdites cellules d'épithélium pigmentaire rétinien.

Les différentes cellules différenciées constituant l'unité de tissu selon l'invention, quel qu'en soit le mode de réalisation, peuvent éventuellement avoir été obtenues à partir de cellules souches pluripotentes, en particulier de cellules souches pluripotentes humaines, ou éventuellement avoir été directement reprogrammées à partir de cellules adultes telles que des fibroblastes ou des cellules mononucléées sanguines périphériques par exemple.

L'unité de tissu selon l'invention peut se présenter sous toute forme en trois dimensions, c'est-à-dire qu'elle peut avoir la forme de tout objet de l'espace. Préférentiellement l'unité de tissu selon l'invention se présente sous la forme d'un ovoïde creux, d'un cylindre creux, d'un sphéroïde creux ou d'une sphère creuse. C'est la couche externe de l'unité de tissu, c'est-à-dire la couche d'épithélium pigmentaire rétinien ou la couche de matrice extracellulaire lorsqu'elle est présente, qui confère sa taille et sa forme à l'unité de tissu selon l'invention. Préférentiellement la plus grande dimension de l'unité de tissu selon l'invention est inférieure à 1cm, encore plus préférentiellement inférieure à 0,5cm. Selon un mode de réalisation adapté et préféré, la plus grande dimension de l'unité de tissu selon l'invention est comprise entre 100 et 1000µm. Elle peut également être comprise entre 200 et 1000µm ou entre 300 et 1000µm. Cette dimension permet d'assurer une implantation aisée dans l'oeil, en particulier par injection, la plus grande dimension ne devant pas être trop importante pour pouvoir être implantée avec une incision préalable de très petite dimension. Plus la plus grande dimension est grande, plus l'incision réalisée dans l'oeil devra être grande et il est important de limiter autant que possible la taille de l'incision pour limiter les risques et l'impact sur le patient traité. Préférentiellement, la plus petite dimension de l'unité de tissu est inférieure à 1000 µm. Selon un mode de réalisation elle est comprise entre 10 et 1000 µm, préférentiellement entre 100 et 400 µm et encore plus préférentiellement entre 200 et 300 µm. Cette plus petite dimension est importante pour la survie du greffon in vitro, en particulier pour favoriser la survie des cellules rétiniennes au sein de l'unité de tissu rétinien et optimiser la réorganisation ainsi que la vascularisation de l'unité de tissu après implantation dans l'oeil.

L'épaisseur de la couche de cellules d'épithélium pigmentaire rétinien dans l'unité de tissu est préférentiellement comprise entre 5µm et 200µm. Lorsque l'unité de tissu selon l'invention comprend une ou plusieurs autres couches de cellules rétiniennes qui ne sont pas des cellules d'épithélium pigmentaire rétinien, cette couche ou ces couches ensemble s'il y en a plusieurs, présente(nt) une épaisseur préférentiellement comprise entre 20µm et 500µm. Lorsqu'une couche externe de matrice extracellulaire est présente dans l'unité de tissu selon l'invention, l'épaisseur de cette couche externe de matrice extracellulaire est préférentiellement comprise entre 30 nm et 500 nm.

La lumière représente préférentiellement entre 10% et 90% du volume de l'unité de tissu selon l'invention.

L'unité de tissu rétinien selon l'invention est particulièrement utile comme greffon implantable dans l'oeil d'un être humain en particulier pour le traitement de maladies de la rétine. La forme, la taille et la constitution de l'unité de cellules de la rétine selon l'invention permettent la survie des cellules au sein de l'unité de tissu avant implantation et la réussite de l'implantation, de la réorganisation et de la vascularisation du greffon une fois implanté dans l'oeil.

Jusqu'à l'implantation, l'unité de tissu peut éventuellement être encapsulée dans une capsule d'hydrogel, dans laquelle elle a été préférentiellement préparée. Dans ce cas la capsule d'hydrogel est préférentiellement éliminée avant implantation dans l'oeil.

Les unités de tissu selon l'invention peuvent être congelées pour leur stockage jusqu'à l'implantation.

### Kit d'implantation

L'invention a également pour objet un kit d'implantation d'au moins une unité de tissu.

Le kit d'implantation selon l'invention comprend au moins :
- entre 1 et 10 000 unités de tissus selon l'invention, les unités de tissus pouvant éventuellement être encapsulées dans une capsule d'hydrogel,
- éventuellement des moyens d'élimination de capsule d'hydrogel, dans le cas où les unités de capsules sont encapsulées dans une capsule d'hydrogel,
- un dispositif d'implantation chirurgical apte à implanter la ou lesdites unité(s) de tissu dans un oeil humain.

Les moyens d'élimination de la capsule d'hydrogel doivent permettre d'éliminer la capsule par hydrolyse, dissolution, perçage et/ou rupture par tout moyen biocompatible, c'est-à-dire non toxique pour les cellules. Préférentiellement, les moyens d'élimination sont choisis parmi les solutions tampons (comme par exemple le tampon phosphate salin appelé également PBS pour « phosphate buffered saline »), un tampon contenant un chélateur d'ions divalents (comme par exemple l'EDTA), les enzymes aptes à lyser l'hydrogel (à choisir en fonction de la nature de l'hydrogel).

Le dispositif d'implantation chirurgical peut être une aiguille ou une canule dont le diamètre interne permet le passage des unités de tissu selon l'invention à greffer, préférentiellement entre 100µm et 1mm et dont le diamètre externe n'est pas trop traumatique pour la structure de l'oeil traité, préférentiellement moins de 2mm.

Le nombre d'unités de tissu selon l'invention présentent dans le dispositif est compris entre 1 et 10 000, préférentiellement entre 10 et 1000. Ce nombre varie en fonction de la maladie de la rétine à traiter et de la taille de la zone de la rétine qui n'est plus fonctionnelle.

Dans le kit d'implantation, les unités de tissu peuvent être hors du dispositif d'implantation et/ou déjà introduites en tout ou partie dans le dispositif d'implantation chirurgical.

Les unités de tissu selon l'invention présentes dans le kit peuvent être congelées hors du dispositif et/ou congelées dans le dispositif d'implantation chirurgical. Dans ce cas, les unités de tissus selon l'invention doivent être décongelées avant utilisation par tout moyen adapté permettant de sauvegarder l'ensemble des propriétés des unités de tissu. Il peut s'agir notamment de protocoles standards de biologie cellulaire utilisant le DMSO, comme antigels, ou ceux appliqués pour la congélation d'embryons de fécondation in vitro utilisant des sucres tel que le saccharose et des alcools comme l'éthylène glycol.

Si les unités de tissu ont été congelées encapsulées dans une capsule d'hydrogel, il convient d'abord de décongeler les unités de tissu encapsulées, puis d'éliminer les capsules d'hydrogel.

### Procédé de préparation

L'invention vise également un procédé de préparation d'une unité de tissu selon l'invention. En particulier le procédé consiste à réaliser au moins une unité de tissu selon l'invention, en réalisant des microcompartiments cellulaires comprenant une capsule d'hydrogel entourant :
- des cellules d'épithélium pigmentaire rétinien différenciées et éventuellement d'autres cellules rétiniennes différenciées, ou
- des cellules souches ou des cellules progénitrices capables de se différencier en cellules de la rétine, au moins en cellules d'épithélium pigmentaire rétinien, ou
- des cellules différenciées destinées à subir dans la capsule :
   * soit une trans-différenciation en cellules de la rétine, au moins en cellules d'épithélium pigmentaire rétinien,
   *soit une reprogrammation dans la capsule de façon à ce qu'elles deviennent des cellules souches pluripotentes induites capables de se différencier en cellules de la rétine, au moins en cellules d'épithélium pigmentaire rétinien.

La capsule est ensuite préférentiellement éliminée afin de permettre aux cellules de l'unité de tissu de s'implanter au niveau de la rétine après une greffe dans l'oeil.

Le procédé de préparation d'une unité de tissu selon l'invention, comprend au moins la mise en oeuvre des étapes qui consistent à :
- produire un microcompartiment cellulaire comprenant, à l'intérieur d'une capsule en hydrogel :
   * préférentiellement au moins des éléments de la matrice extracellulaire, sécrétés par les cellules ou apportés par l'opérateur, préférentiellement au moins une partie de la matrice extracellulaire est apportée en plus de la matrice extracellulaire naturellement sécrétée par les cellules,
   * des cellules aptes à se différencier au moins en cellules d'épithélium pigmentaire rétinien, ou au moins des cellules d'épithélium pigmentaire rétinien différenciées,
   les cellules aptes à se différencier au moins en cellules d'épithélium pigmentaire rétinien étant choisies parmi :
   * des cellules souches capables de se différencier en cellules de la rétine, au moins en cellules d'épithélium pigmentaire rétinien,
   * des cellules progénitrices capables, de se différencier en cellules de la rétine, au moins en cellules d'épithélium pigmentaire rétinien,
   * des cellules différenciées capables de subir une trans-différenciation en cellules de la rétine, au moins en cellules d'épithélium pigmentaire rétinien,
   * des cellules différenciées capables de subir une reprogrammation de façon à ce qu'elles deviennent des cellules souches pluripotentes induites capables de se différencier en cellules de la rétine, au moins en cellules d'épithélium pigmentaire rétinien
- si les cellules introduites dans le microcompartiment sont des cellules aptes à se différencier au moins en cellules d'épithélium pigmentaire rétinien : induire la différenciation cellulaire au sein du microcompartiment cellulaire, de manière à obtenir au moins des cellules d'épithélium pigmentaire rétinien et éventuellement d'autres cellules rétiniennes,
- éliminer les capsules d'hydrogel, par hydrolyse, dissolution, perçage et/ou rupture, pour récupérer les cellules d'épithélium pigmentaire rétinien et les éventuelles autres cellules rétiniennes sous forme d'une unité de tissu rétinien tridimensionnelle creuse comprenant, organisée autour d'une lumière interne, au moins une couche d'épithélium pigmentaire rétinien dont la face basale de chaque cellule d'épithélium pigmentaire rétinien est positionnée vers l'extérieur et la face apicale vers la lumière.

Avantageusement, l'encapsulation totale ou partielle dans l'hydrogel et l'apport de matrice extracellulaire combinés, est un moyen adapté pour permettre la polarisation des cellules de l'épithélium pigmentaire rétinien. En effet, la polarisation desdites cellules peut être obtenue par déposition d'une couche de matrice sur la face interne des capsules d'hydrogel qui positionne la face basale des cellules, le tissus s'organise autour de la lumière en suivant cette indication de polarité (tel qu'illustré sur la Figure 9 qui montre qu'une couche de matrice extracellulaire ancrée à la coque d'alginate induit la polarisation des cellules comme en témoigne l'aplatissement des tissus contre l'alginate en raison de la résistance à la traction élevée du gel dictant la forme du tissu).Préférentiellement l'hydrogel utilisé est biocompatible, c'est-à-dire qu'il n'est pas toxique pour les cellules. La capsule d'hydrogel doit permettre la diffusion d'oxygène et de nutriment pour alimenter les cellules contenues dans le microcompartiment et permettre leur survie. Selon un mode de réalisation, la capsule comprend de l'alginate. Elle peut être constituée exclusivement d'alginate. L'alginate peut être en particulier un alginate de sodium, composé à 80% d'α-L-guluronate et 20% de β-D-mannuronate, avec une masse moléculaire moyenne de 100 à 400 kDa et une concentration totale comprise entre 0,5 et 5% en masse.

La capsule d'hydrogel permet de protéger les cellules du milieu extérieur, de limiter la prolifération incontrôlée des cellules, et leur différenciation contrôlée en cellules rétiniennes, au moins en cellules d'épithélium pigmentaire rétinien. Une capsule entoure très préférentiellement une seule unité de tissu selon l'invention et chaque unité de tissu est entourée par une seule capsule d'hydrogel.

Une fois que l'unité de tissu rétinien selon l'invention est obtenue, c'est-à-dire lorsque les cellules sont différenciées en cellules rétiniennes dont au moins une couche de cellules d'épithélium pigmentaire rétinien, et que la forme et la taille sont celles recherchées, la capsule est éliminée. L'élimination de la capsule peut être réalisée à la fin du procédé ou plus tard dans le temps avant l'implantation dans l'oeil. L'élimination de la capsule peut être réalisée par hydrolyse, dissolution, perçage et/ou rupture par tout moyen biocompatible, c'est-à-dire non toxique pour les cellules. Par exemple, l'élimination peut être réalisée en utilisant un tampon phosphate salin, un chélateur d'ions divalents, une enzyme comme l'alginate lyase si l'hydrogel comprend de l'alginate et/ou la microdissection laser. L'élimination de l'hydrogel étant totale, l'unité de tissu selon l'invention est dépourvue d'hydrogel lorsqu'elle est implantée dans l'oeil.

Tout procédé de production de microcompartiments cellulaires contenant à l'intérieur d'une capsule d'hydrogel au moins des cellules d'épithéliume pigmentaire rétinien ou des cellules susceptibles de donner au moins des cellules d'épithélium pigmentaire rétinien et éventuellement de la matrice extracellulaire et/ou d'autres cellules rétiniennes que des cellules d'épithélium pigmentaire rétinien ou des cellules susceptibles de donner au moins des cellules rétiniennes autres que des cellules d'épithélium pigmentaire rétinien, peut être utilisé. Une méthode adaptée est notamment décrite dans la demande WO2018/096277. Dans un mode de réalisation particulier, l'étape de production d'un microcompartiment cellulaire du procédé de préparation selon l'invention, comprend les étapes consistant à :
- incuber des cellules souches pluripotentes dans un milieu de culture, préférentiellement un milieu de culture basé sur le DMEM ou le DMEM-F12, du FGF-2 ou une molécule reproduisant son action sur la cellule, du TGF-beta ou une molécule reproduisant son action sur la cellule.
- mélanger les cellules souches pluripotentes avec une matrice extracellulaire,
- encapsuler le mélange dans une couche d'hydrogel.

Les cellules encapsulées pour la préparation d'unité de tissu selon l'invention sont préférentiellement choisies parmi :
- des cellules aptes à se différencier au moins en cellules d'épithélium pigmentaire rétinien, ces cellules étant :
   * soit des cellules souches capables de se différencier en cellules de la rétine, au moins en cellules d'épithélium pigmentaire rétinien, préférentiellement des cellules souches embryonnaires ou des cellules souches pluripotentes induites, très préférentiellement des cellules souches pluripotentes induites, et/ou,
   * soit des cellules progénitrices capables, de se différencier en cellules de la rétine, au moins en cellules d'épithélium pigmentaire rétinien,
- et/ou des cellules d'épithélium pigmentaire rétinien différenciées et éventuellement des cellules rétiniennes différenciées autres que des cellules d'épithélium pigmentaire rétinien,
- et/ou des cellules différenciées capables de subir une trans-différenciation en cellules de la rétine, au moins en cellules d'épithélium pigmentaire rétinien,
- et/ou des cellules différenciées capables de subir une reprogrammation de façon à ce qu'elles deviennent des cellules souches pluripotentes induites capables de se différencier en cellules de la rétine, au moins en cellules d'épithélium pigmentaire rétinien.

Les cellules encapsulées peuvent être immuno-compatibles avec la personne destinée à recevoir l'unité de tissu, pour éviter tout risque de rejet. Dans un mode de réalisation, les cellules encapsulées ont été préalablement prélevées chez la personne dans laquelle la ou les unités de tissus doivent être implantées.

La différenciation en cellules d'épithélium pigmentaire rétinien peut être réalisée par tout procédé adapté. Il peut notamment s'agir d'une méthode connue comme une des méthodes décrites dans Leach et al. (« Concise Review: Making Stem Cells Retinal: Methods for Deriving Retinal Pigment Epithelium and Implications for Patients With Ocular Disease », Stem Cells 2015;33:2363-2373). Le milieu de base peut être du DMEM (« Dulbecco's modified Eagle's medium ») ou du DMEMF12 qui peut être complémenté par du KSR-XF (« KnockOut DMEM medium ») ou du N-2 et/ou du B27, 1% de GlutaMax, et 1% de solution d'amino-acide non-essentiels. L'induction peut aussi être obtenue avec la séquence telle que publiée dans Choudhary et al. (« Directing Differentiation of Pluripotent Stem Cells Toward Retinal Pigment Epithelium Lineage » STEM CELLS TRANSLATIONAL MEDICINE 2016;5:1-12)

La différenciation en cellules rétiniennes autres que des cellules d'épithélium pigmentaire rétinien peut être réalisée par tout procédé adapté. Il peut notamment s'agir d'une méthode connue comme la méthode décrite dans Barnea-Cramer et al. (« Function of human pluripotent stem cell-derived photoreceptor progenitors in blind mice » Nature, Scientific Reports, publié le 13 juillet 2016). La différenciation peut donc être réalisée avec DMEMF12 qui peut être complémenté par du KSR-XF (KnockOut DMEM medium) ou du N-2 et/ou du B27, 1% de GlutaMax, et 1% de solution d'amino-acide non-essentiels.

Dans un mode de réalisation particulier, l'étape d'induction de la différenciation cellulaire du procédé de préparation selon l'invention, comprend les étapes consistant à :
- cultiver le microcompartiment dans un milieu de culture de cellules pluripotentes jusqu'à obtenir au moins 10 cellules préférentiellement 100 cellules,
- cultiver le microcompartiment dans un milieu de culture DMEMF12 complémenté par N-2 et B27, 1% de GlutaMax, et 1% de solution d'amino-acide non-essentiels et LDN193189 et SB431542 et 20 µg/ml d'insuline humaine,
- cultiver le microcompartiment dans un milieu de culture DMEMF12 complémenté par N-2 et B27, 1% de GlutaMax, et 1% de solution d'amino-acide non-essentiels et LDN193189 et SB431542,
- cultiver le microcompartiment dans un milieu de culture DMEMF12 complémenté par N-2 et B27, 1% de GlutaMax, et 1% de solution d'amino-acide non-essentiels et 10 ng/ml de human BDNF, 10 ng/ml de human CNTF, 2 µM d'acide retinoïque et 10 µ M de DAPT. Préférentiellement les microcompartiments sont cultivés pendant au moins 18 jours, préférentiellement entre 18 et 50 jours.

Dans un mode de réalisation d'une unité de tissu comprenant une couche d'épithélium pigmentaire rétinien et au moins une couche d'autres cellules rétiniennes, le procédé consiste à co-encapsuler des cellules d'épithélium pigmentaire rétinien et les autres cellules. Préférentiellement, les cellules issues de quelques jours de chaque différenciation sont co-encapsulées pour former une structure contenant un d'épithélium pigmentaire rétinien et une de rétine neurale. Les cellules sont ensuite maturées dans la capsule pendant 5 à 50 jours, plus préférentiellement 10 à 25 jours avant d'obtenir une unité de tissu selon l'invention.

La lumière est générée au moment de la formation de l'unité de tissu en trois dimensions, par les cellules qui se multiplient et se développent. La lumière peut contenir un liquide et en particulier le milieu de culture utilisé pour la mise en oeuvre du procédé.

Le procédé selon l'invention peut comprendre une étape d'amplification des cellules d'épithélium pigmentaire rétinien, dans le microcompartiment.

Un mode de réalisation d'un microcompartiement 20 comprenant une unité de tissu rétinien tridimensionnelle creuse 10 selon l'invention, est représenté sur la figure 3. Dans ce mode de réalisation, le microcompartiment est constituée exclusivement :
- d'une couche d'hydrogel 22, et
- d'une unité de tissu 10, constituée :
   * d'une lumière interne 14,
   * d'une couche d'épithélium pigmentaire rétinien 12 organisée autour de la lumière interne, les faces basales B des cellules épithéliales pigmentaires rétiniennes étant dirigées vers l'extérieur de l'unité de tissu, et les faces apicales A des cellules épithéliales pigmentaires rétiniennes étant dirigées vers la lumière interne,
   * d'une couche de matrice extracellulaire 16 disposée autour de la couche de cellules d'épithélium pigmentaire rétinien, au niveau des faces basales desdites cellules d'épithélium pigmentaire rétinien.

Un autre mode de réalisation d'un microcompartiement 20 comprenant une unité de tissu rétinien tridimensionnelle creuse 10 selon l'invention, est représenté sur la figure 4. Dans ce mode de réalisation, le microcompartiment est constitué exclusivement :
- d'une couche d'hydrogel 22, et
- d'une unité de tissu 10, constituée :
   * d'une lumière interne 14,
   * d'une couche 18 de cellules rétiniennes autres que des cellules épithéliales pigmentaires rétiniennes, de façon préférée une couche de rétine neurale,
   * d'une couche d'épithélium pigmentaire rétinien 12 organisée autour de la lumière interne, les faces basales B des cellules épithéliales pigmentaires rétiniennes étant dirigées vers l'extérieur de l'unité de tissu, et les faces apicales A des cellules épithéliales pigmentaires rétiniennes étant dirigées vers la lumière interne,
   * d'une couche de matrice extracellulaire 16 disposée autour de la couche de cellules d'épithélium pigmentaire rétinien, au niveau des faces basales desdites cellules d'épithélium pigmentaire rétinien.

Après l'étape de différenciation, à tout moment avant l'implantation des unités de tissu dans l'oeil, le procédé selon l'invention peut comprendre une étape consistant à vérifier le phénotype des cellules contenues dans la capsule. Cette vérification peut être réalisée en identifiant l'expression de RPE65 par l'épithélium pigmentaire en position externe des unités de tissu, dans un certain mode de réalisation et en particulier pour le cas d'éléments tissus contenant des éléments de la rétine neurale de recoverin en position interne des unités de tissu, côté lumière, exprimés par l'ensemble des photorécepteurs et de rhodopsin/PDE6beta pour les bâtonnets, dans un certain mode de réalisation le lumen peut contenir de la vitrosine et de l'opticine.

Le procédé selon l'invention peut comprendre une étape de congélation des microcompartiments contenant les unités de tissu selon l'invention avant élimination de la couche d'hydrogel ou de congélation des unités de tissu après l'étape d'élimination de la capsule d'hydrogel. La congélation est préférentiellement réalisée à une température comprise entre -190°C et -80°C.

Les microcompartiments contenant les unités de tissu selon l'invention avant élimination de la couche d'hydrogel ou les unités de tissu après l'étape d'élimination de la capsule d'hydrogel, peuvent être stockés dans les conditions suivantes entre +4°C et la température ambiante. Les unités de tissu selon l'invention peuvent également être utilisées directement après mise en oeuvre du procédé selon l'invention, sans stockage.

Le procédé de préparation selon l'invention, avant ou après décongélation éventuelle des microcompartiments contenant les unités de tissu avant élimination de la couche d'hydrogel ou les unités de tissu, peut également comprendre une étape supplémentaire consistant à charger un dispositif d'implantation chirurgical avec au moins une unité de tissu selon l'invention, préférentiellement entre 10 et 1000 unités de tissu, encore plus préférentiellement entre 10 et 100 unités de tissu.

### Implantation d'unités de tissu dans l'oeil

L'unité de tissu tridimensionnelle creuse, comprenant, organisée autour d'une lumière interne, au moins une couche de cellules d'épithélium pigmentaire rétinien dont la face basale de chaque cellule d'épithélium pigmentaire rétinien est positionnée vers l'extérieur et la face apicale vers la lumière interne, peut être utilisée dans le traitement d'une maladie de la rétine, notamment chez un patient en ayant besoin. Par traitement on entend un traitement préventif, curatif ou symptomatique, c'est-à-dire tout acte destiné à améliorer la vue d'une personne de façon temporaire ou définitive, et préférentiellement également à éradiquer la maladie et/ou arrêter ou retarder la progression de la maladie et/ou favoriser la régression de la maladie.

En effet les unités de tissu selon l'invention peuvent être utilisées pour le traitement de maladies de la rétine chez l'homme, en particulier de maladies dégénératives de la rétine, et préférentiellement une maladie choisie parmi la dégénérescence maculaire liée à l'âge, la rétinopathie diabétique, les rétinopathies liées à un traumatisme de l'oeil et les rétinopathies héréditaires.

Le traitement consiste à implanter, à greffer les unités de tissu selon l'invention dans l'oeil, au niveau de la rétine, et en particulier au niveau la membrane de Bruch c'est-à-dire entre la membrane de Bruch et la rétine neurale. Un dispositif d'implantation chirurgical adapté à une implantation dans l'oeil est très préférentiellement utilisé. Il peut s'agir notamment d'aiguilles, de canules ou autre dispositif permettant de déposer les unités de tissus comme par exemple ceux utilisés pour l'implantation de stents dans les artères ou de micro implants chirurgicaux. L'implantation peut être réalisée notamment par la mise en oeuvre des étapes consistant à :
- pénétrer ou inciser la rétine avec le dispositif d'implantation chirurgicale dans la zone de traitement,
- injecter les unités de tissus sous la rétine, préférentiellement au niveau de la membrane de Bruch, c'est-à-dire entre la membrane de Bruch et la rétine neurale,
- retirer le dispositif d'implantation chirurgicale en empêchant le refoulement dans le corps vitreux des cellules.

Dans un mode de réalisation, lors d'une même implantation, entre 1 et 10000 unités de tissu selon l'invention sont implantées.

Si nécessaire, il est possible de réaliser plusieurs implantations simultanées ou successives dans différentes zones de la rétine, préférentiellement au niveau de la membrane de Bruch, en particulier dans le cas où plusieurs zones séparées sont touchées par la maladie ou si la zone sur laquelle il faut réaliser la greffe, est trop étendue pour ne réaliser une greffe qu'à un endroit.

De même, sur une même zone, si une seule greffe ne suffit pas, plusieurs implantations peuvent être de nouveau réalisées sur la même zone, dans un laps de temps plus ou moins important.

L'implantation d'unités de tissu selon l'invention permet aux patients atteints de maladies de la rétine, et en particulier de maladies dégénératives de la rétine de retrouver au moins partiellement la vue.

L'invention est à présent illustrée par des résultats illustrés sur les Figures 5 à 8.

Les tissus d'épithélium rétinien illustrés sur ces différentes figures ont été obtenus par la mise en oeuvre d'un procédé comprenant les étapes consistant à :
- produire un microcompartiment cellulaire comprenant, à l'intérieur d'une capsule en hydrogel :
   * des éléments de la matrice extracellulaire apportés par l'opérateur,
   * des cellules d'épithélium rétinal pigmenté,

On peut obtenir ces cellules d'épithélium rétinal pigmenté de la façon suivante :
- cultiver des cellules souches induites à la pluripotence au sein i) d'une boite de pétri jusqu'à obtenir des colonies contenant plusieurs dizaines de cellules ii) du microcompartiment dans un milieu de culture de cellules pluripotentes, jusqu'à obtenir dans le microcompartiment au moins 10 cellules préférentiellement 100 cellules,
- cultiver : i) les colonies ii) le microcompartiment dans un milieu de culture DMEMF12 complémenté par N-2 et B27, 1% de GlutaMax, et 1% de solution d'amino-acide non-essentiels et LDN193189 et SB431542 et 20 µg/ml d'insuline humaine,
- cultiver : i) les colonies ii) le microcompartiment dans un milieu de culture DMEMF12 complémenté par N-2 et B27, 1% de GlutaMax, et 1% de solution d'amino-acide non-essentiels et LDN193189 et SB431542,
- cultiver : i) les colonies ii) le microcompartiment dans un milieu de culture DMEMF12 complémenté par N-2 et B27, 1% de GlutaMax, et 1% de solution d'amino-acide non-essentiels et 10 ng/ml de human BDNF, 10 ng/ml de human CNTF, 2 µM d'acide retinoïque et 10 µ M de DAPT.

Sur la figure 5, la capsule obtenue par encapsulation de cellules épithéliales pigmentées de la rétine issues de la différenciation de cellules induites ont proliféré au sein du microcompartiment pour s'organiser sous forme d'un unité de tissu polarisé. Sur la figure 5 on voit bien la formation d'un épithélium monostratifié pavimenteux pigmenté (flèche noire) mature (typiquement permis par la formation de jonction serrées flèche blanche) typique de la structure tissulaire des cellules épithéliales pigmentées de la rétine in vivo.

Sur la figure 6, les capsules obtenues par encapsulation de cellules épithéliales pigmentées de la rétine issues de la différenciation de cellules induites à la pluripotence montrent la structuration en feuillet épithélial pigmenté quel que soit le nombre de cellules dans le tissus (de l'ordre de la centaine de cellules à gauche, taille : 50µm, insert A, et de l'ordre du millier de cellules à droite, taille : 250µm, insert B) typique de la structure tissulaire des cellules épithéliales pigmentées de la rétine in vivo. Plus particulièrement on voit sur la figure 6 que les cellules sont organisées en sphéroïde creux (insert B) ovoïde creux (insert E) selon une polarisation apico-basale caractérisée en ce que la face apicale est positionnée vers l'intérieur de l'unité de tissus et la face basale vers l'extérieur de l'unité de tissus située ici en contact avec la couche d'alginate. En particulier les pigments intracellulaires sont situés en face apicale des cellules (insert D, lumière transmise, flèche blanche) avec une organisation basale des noyaux (insert B, DAPI, flèche blanche) correspondant à la topologie rencontrée in vivo.

Sur la figure 7 les capsules obtenues par encapsulation de cellules épithéliales pigmentées de la rétine issues de la différenciation de cellules induites à la pluripotence, ont été amplifiées par 10 en 4 semaines (figures 7a et 7b) et 4 fois en 4 semaines (figures 7c et 7d). Ces figures illustrent que les unités de tissu rétiniens selon l'invention peuvent avoir des densités cellulaires variables.

Enfin, sur les images séquentielles de la figure 8, plusieurs unités de tissu rétinien selon l'invention (sans capsule d'hydrogel) ont été disposées sur un substrat simulant la matrice extracellulaire du fond de l'oeil (ici matrigel simulant la membrane de Brusch). On constate que les unités de tissu rétinien (flèches noires) sont capables grâce au positionnement de leur face basale coté extérieur de s'attacher au substrat simulant la matrice extracellulaire du fond de l'oeil en émettant des cellules adhérentes au substrat (flèches blanches) qui migrent (flèches grises) pour recouvrir le substrat et forment une monocouche (insert en bas à droite correspondant à 122 heures de culture).

## Revendications

1. Unité de tissu rétinien tridimensionnelle creuse, comprenant, organisée autour d'une lumière interne, au moins une couche de cellules d'épithélium pigmentaire rétinien humaines vivantes différenciées dont la face basale de chaque cellule d'épithélium pigmentaire rétinien est positionnée vers l'extérieur et la face apicale vers la lumière interne.

2. Unité de tissu rétinien selon la revendication 1, **caractérisée en ce qu'**elle comprend également une couche externe de matrice extracellulaire située au niveau de la face basale des cellules d'épithélium pigmentaire rétinien.

3. Unité de tissu rétinien selon l'une des précédentes revendications, **caractérisée en ce qu'**elle se présente sous la forme d'un ovoïde creux, d'un cylindre creux, d'un sphéroïde creux ou d'une sphère creuse.

4. Unité de tissu rétinien selon la précédence revendication, **caractérisée en ce que** sa plus grande dimension est comprise entre 100 et 1000µm.

5. Unité de tissu rétinien selon la précédente revendication, **caractérisée en ce que** sa plus petite dimension est comprise entre 10 et 1000µm.

6. Unité de tissu rétinien selon l'une des précédentes revendications, **caractérisée en ce que** les cellules d'épithélium pigmentaire rétinien juxtaposées sont liées entre elles sur leurs faces latérales par des jonctions serrées.

7. Unité de tissu rétinien selon l'une des précédentes revendications, **caractérisée en ce qu'**elle comprend également, au niveau de la face apicale des cellules d'épithélium pigmentaire rétinien, organisée autour de la lumière interne, au moins une couche de cellules rétiniennes humaines vivantes différenciées autres que des cellules d'épithélium pigmentaire rétinien.

8. Unité de tissu rétinien selon la précédente revendication, **caractérisée en ce que** les cellules rétiniennes humaines vivantes différenciées autres que des cellules d'épithélium pigmentaire rétinien sont choisies parmi les bâtonnets, les cônes, les cellules ganglionnaires, les cellules amacrines, les cellules bipolaires et les cellules horizontales.

9. Unité de tissu rétinien selon l'une des précédentes revendications, **caractérisée en ce qu'**elle contient de 10 à 100 000 cellules rétiniennes.

10. Unité de tissu rétinien selon l'une des précédentes revendications, **caractérisée en ce que** les cellules d'épithélium pigmentaire rétinien et/ou les éventuelles autres cellules rétiniennes ont été obtenues à partir de cellules souches induites à la pluripotence (IPS).

11. Unité de tissu rétinien selon l'une des précédentes revendications, **caractérisée en ce qu'**elle est encapsulée dans une capsule d'hydrogel.

12. Procédé de préparation d'une unité de tissu rétinien selon l'une des revendications 1 à 11, comprenant les étapes consistant à :
- produire un microcompartiment cellulaire comprenant, à l'intérieur d'une capsule en hydrogel :
* éventuellement au moins des éléments de la matrice extracellulaire, sécrétés par les cellules ou ajoutés,
* des cellules aptes à se différencier au moins en cellules d'épithélium pigmentaire rétinien, ou au moins des cellules d'épithélium pigmentaire rétinien différenciées,
les cellules aptes à se différencier au moins en cellules d'épithélium pigmentaire rétinien étant choisies parmi :
* des cellules souches capables de se différencier en cellules de la rétine, au moins en cellules d'épithélium pigmentaire rétinien,
* des cellules progénitrices capables, de se différencier en cellules de la rétine, au moins en cellules d'épithélium pigmentaire rétinien,
* des cellules différenciées capables de subir une trans-différenciation en cellules de la rétine, au moins en cellules d'épithélium pigmentaire rétinien,
* des cellules différenciées capables de subir une reprogrammation de façon à ce qu'elles deviennent des cellules souches pluripotentes induites capables de se différencier en cellules de la rétine, au moins en cellules d'épithélium pigmentaire rétinien
- si les cellules introduites dans le microcompartiment sont des cellules aptes à se différencier au moins en cellules d'épithélium pigmentaire rétinien : induire la différenciation cellulaire au sein du microcompartiment cellulaire, de manière à obtenir au moins des cellules d'épithélium pigmentaire rétinien et éventuellement d'autres cellules rétiniennes,
- éliminer les capsules d'hydrogel par hydrolyse, dissolution, perçage et/ou rupture pour récupérer les cellules d'épithélium pigmentaire rétinien et les éventuelles autres cellules rétiniennes sous forme d'une unité de tissu rétinien tridimensionnelle creuse,

13. Procédé selon la précédente revendication, **caractérisé en ce qu'**il comprend une étape d'amplification des cellules d'épithélium pigmentaire rétinien

14. Procédé selon l'une des revendications 12 ou 13, **caractérisé en ce que** les cellules aptes à se différencier au moins en cellules d'épithélium pigmentaire rétinien sont des cellules souches pluripotentes.

15. Procédé selon la précédente revendication, **caractérisé en ce que** les cellules souches pluripotentes sont des cellules souches induites à la pluripotence (IPS).

16. Procédé de préparation d'unité de tissu rétinien selon l'une des revendications 12 à 15, **caractérisé en ce qu'**il comprend une étape supplémentaire consistant à charger un dispositif d'implantation chirurgical adapté à une injection dans l'oeil, avec ladite unité de tissu.

17. Kit d'implantation dans l'oeil d'unités de tissu selon l'une des revendications 1 à 11, **caractérisé en ce qu'**il comprend :
- entre 1 et 10 000 unités de tissu selon l'une des revendications 1 à 11,
- un dispositif d'implantation chirurgical apte à implanter la ou lesdites unité(s) tissu(s) dans un oeil humain.

18. Kit d'implantation dans l'oeil d'unités de tissu selon la revendication 17 **caractérisé en ce qu'**il comprend :
- entre 1 et 10 000 unités de tissu selon la revendication 11,
- des moyens d'élimination de capsule d'hydrogel,
- un dispositif d'implantation chirurgical apte à implanter la ou lesdites unité(s) tissu(s) dans un oeil humain.

## Patentansprüche

1. Hohle dreidimensionale Netzhautgewebeeinheit, umfassend, um ein inneres Lumen herum organisiert, mindestens eine Schicht differenzierter lebender menschlicher Netzhautpigmentepithelzellen, wobei die basale Fläche jeder Netzhautpigmentepithelzelle nach außen und die apikale Fläche zu dem inneren Lumen positioniert ist.

2. Netzhautgewebeeinheit nach Anspruch 1,
**dadurch gekennzeichnet, dass** sie auch eine äußere Schicht einer extrazellulären Matrix umfasst, die sich an der basalen Fläche der Netzhautpigmentepithelzellen befindet.

3. Netzhautgewebeeinheit nach einem der vorstehenden Ansprüche, **dadurch gekennzeichnet, dass** sie in der Form eines hohlen Ovoids, eines hohlen Zylinders, eines hohlen Sphäroids oder einer hohlen Kugel vorliegt.

4. Netzhautgewebeeinheit nach dem vorstehenden Anspruch,
**dadurch gekennzeichnet, dass** ihre größte Abmessung zwischen 100 und 1000 µm liegt.

5. Netzhautgewebeeinheit nach dem vorstehenden Anspruch,
**dadurch gekennzeichnet, dass** ihre kleinste Abmessung zwischen 10 und 1000 µm liegt.

6. Netzhautgewebeeinheit nach einem der vorstehenden Ansprüche, **dadurch gekennzeichnet, dass** nebeneinanderliegende Netzhautpigmentepithelzellen an ihren seitlichen Flächen durch Tight Junctions miteinander verbunden sind.

7. Netzhautgewebeeinheit nach einem der vorstehenden Ansprüche, **dadurch gekennzeichnet, dass** sie ferner an der apikalen Fläche der Netzhautpigmentepithelzellen um das innere Lumen herum organisiert mindestens eine Schicht differenzierter lebender menschlicher Netzhautzellen außer den Netzhautpigmentepithelzellen umfasst.

8. Netzhautgewebeeinheit nach dem vorstehenden Anspruch,
**dadurch gekennzeichnet, dass** differenzierte lebende menschliche Netzhautzellen außer den Netzhautpigmentepithelzellen aus Stäbchen, Zapfen, Lymphknotenzellen, Amakrinzellen, bipolaren Zellen und horizontalen Zellen ausgewählt sind.

9. Netzhautgewebeeinheit nach einem der vorstehenden Ansprüche, **dadurch gekennzeichnet, dass** sie 10 bis 100.000 Netzhautzellen enthält.

10. Netzhautgewebeeinheit nach einem der vorstehenden Ansprüche, **dadurch gekennzeichnet, dass** die Netzhautpigmentepithelzellen und/oder die optionalen anderen Netzhautzellen aus induzierten pluripotenten Stammzellen (IPS) erhalten wurden.

11. Netzhautgewebeeinheit nach einem der vorstehenden Ansprüche, **dadurch gekennzeichnet, dass** sie in einer Hydrogelkapsel eingekapselt ist.

12. Verfahren zum Herstellen einer Netzhautgewebeeinheit nach einem der Ansprüche 1 bis 11, umfassend die Schritte bestehend aus:
- Erzeugen eines zellulären Mikrokompartiments, umfassend, im Inneren einer Hydrogelkapsel:
* optional mindestens Elemente der extrazellulären Matrix, die durch die Zellen abgesondert oder zugesetzt sind,
* Zellen, die geeignet sind, sich mindestens in Netzhautpigmentepithelzellen zu differenzieren, oder mindestens differenzierte Netzhautpigmentepithelzellen, wobei die Zellen, die geeignet sind, sich mindestens in Netzhautpigmentepithelzellen zu differenzieren, ausgewählt sind aus:
* Stammzellen, die in der Lage sind, sich in Netzhautzellen, mindestens in Netzhautpigmentepithelzellen, zu differenzieren,
* Vorläuferzellen, die in der Lage sind, sich in Netzhautzellen, mindestens in Netzhautpigmentepithelzellen, zu differenzieren,
*differenzierte Zellen, die in der Lage sind, eine Transdifferenzierung in Netzhautzellen, mindestens in Netzhautpigmentepithelzellen, zu erfahren,
* differenzierte Zellen, die in der Lage sind, eine Umprogrammierung zu erfahren, sodass sie zu induzierten pluripotenten Stammzellen werden, die in der Lage sind, sich in Netzhautzellen, mindestens in Netzhautpigmentepithelzellen, zu differenzieren
- falls die in das Mikrokompartiment eingebrachten Zellen Zellen sind, die geeignet sind, sich mindestens in Netzhautpigmentepithelzellen zu differenzieren: Induzieren der Zelldifferenzierung innerhalb des zellulären Mikrokompartiments, um mindestens Netzhautpigmentepithelzellen und optional andere Netzhautzellen zu erhalten,
- Entfernen der Hydrogelkapseln durch Hydrolyse, Auflösung, Durchbohren und/oder Aufbrechen zum Gewinnen der Netzhautpigmentepithelzellen und der optionalen anderen Netzhautzellen in Form einer hohlen dreidimensionalen Netzhautgewebeeinheit,

13. Verfahren nach dem vorstehenden Anspruch,
**dadurch gekennzeichnet, dass** es einen Schritt einer Amplifikation von Netzhautpigmentepithelzellen umfasst.

14. Verfahren nach einem der Ansprüche 12 oder 13,
**dadurch gekennzeichnet, dass** die Zellen, die geeignet sind, sich mindestens in Netzhautpigmentepithelzellen zu differenzieren, pluripotente Stammzellen sind.

15. Verfahren nach dem vorstehenden Anspruch,
**dadurch gekennzeichnet, dass** die pluripotenten Stammzellen induzierte pluripotente Stammzellen (IPS) sind.

16. Verfahren zum Herstellen einer Netzhautgewebeeinheit nach einem der Ansprüche 12 bis 15, **dadurch gekennzeichnet, dass** es einen zusätzlichen Schritt umfasst, der darin besteht, eine chirurgische Implantationsvorrichtung, die für eine Injektion in das Auge geeignet ist, mit der Gewebeeinheit zu beladen.

17. Kit zum Implantieren in das Auge von Gewebeeinheiten nach einem der Ansprüche 1 bis 11, **dadurch gekennzeichnet, dass** es umfasst:
- zwischen 1 und 10.000 Gewebeeinheiten nach einem der Ansprüche 1 bis 11,
- eine chirurgische Implantationsvorrichtung, die geeignet ist, die Gewebeeinheit(en) in ein menschliches Auge zu implantieren.

18. Kit zum Implantieren von Gewebeeinheiten in das Auge nach Anspruch 17, **dadurch gekennzeichnet, dass** es umfasst:
- zwischen 1 und 10.000 Gewebeeinheiten nach Anspruch 11,
- Mittel zum Entfernen von Hydrogelkapseln,
- eine chirurgische Implantationsvorrichtung, die geeignet ist, die Gewebeeinheit(en) in ein menschliches Auge zu implantieren.

## Claims

1. A hollow three-dimensional retinal tissue unit comprising, organized around an inner cavity, at least one layer of differentiated living human retinal pigment epithelium cells, with the basal side of each retinal pigment epithelium cell pointing outwards and the apical side pointing toward the inner cavity.

2. The retinal tissue unit according to claim 1, **characterized in that** it also comprises an outer layer of extracellular matrix located on the basal side of the retinal pigment epithelium cells.

3. The retinal tissue unit according to one of the preceding claims, **characterized in that** it is in the form of a hollow ovoid, a hollow cylinder, a hollow spheroid or a hollow sphere.

4. The retinal tissue unit according to the preceding claim,
**characterized in that** its largest dimension is between 100 and 1000 µm.

5. The retinal tissue unit according to the preceding claim,
**characterized in that** its smallest dimension is between 10 and 1000 µm.

6. The retinal tissue unit according to one of the preceding claims, **characterized in that** the juxtaposed retinal pigment epithelium cells are connected to one another on their lateral sides by tight junctions.

7. The retinal tissue unit according to one of the preceding claims, **characterized in that** it also comprises, at the apical side of the retinal pigment epithelium cells, organized around the inner cavity, at least one layer of differentiated living human retinal cells other than retinal pigment epithelium cells.

8. The retinal tissue unit according to the preceding claim,
**characterized in that** differentiated living human retinal cells other than retinal pigment epithelium cells are selected from rods, cones, ganglion cells, amacrine cells, bipolar cells and horizontal cells.

9. The retinal tissue unit according to one of the preceding claims, **characterized in that** it contains from 10 to 100,000 retinal cells.

10. The retinal tissue unit according to one of the preceding claims, **characterized in that** the retinal pigment epithelium cells and/or any other retinal cells have been obtained from induced pluripotent stem cells (IPS).

11. The retinal tissue unit according to one of the preceding claims, **characterized in that** it is encapsulated in a hydrogel capsule.

12. A method for preparing a retinal tissue unit according to one of claims 1 to 11, comprising the steps of:
- producing a cellular microcompartment comprising, inside a hydrogel capsule:
* optionally at least extracellular matrix elements, secreted by the cells or added,
* cells capable of differentiating into at least retinal pigment epithelium cells, or at least differentiated retinal pigment epithelium cells,
the cells capable of differentiating into at least retinal pigment epithelium cells being selected from:
* stem cells capable of differentiating into retinal cells, at least into retinal pigment epithelium cells,
* progenitor cells capable of differentiating into retinal cells, at least into retinal pigment epithelium cells,
* differentiated cells capable of undergoing trans-differentiation into retinal cells, at least into retinal pigment epithelium cells,
* differentiated cells capable of undergoing reprogramming so that they become induced pluripotent stem cells capable of differentiating into retinal cells, at least into retinal pigment epithelium cells
- if the cells introduced into the microcompartment are cells capable of differentiating into at least retinal pigment epithelium cells: inducing cell differentiation within the cell microcompartment, so as to obtain at least retinal pigment epithelium cells and optionally other retinal cells,
- removing hydrogel capsules by hydrolysis, dissolution, piercing and/or rupture to recover retinal pigment epithelium cells and any other retinal cells in the form of a hollow, three-dimensional retinal tissue unit,

13. The method according to the preceding claim, **characterized in that** it comprises a step of amplifying the retinal pigment epithelium cells

14. The method according to one of claims 12 or 13, **characterized in that** the cells capable of differentiating at least into retinal pigment epithelium cells are pluripotent stem cells.

15. The method according to the preceding claim, **characterized in that** the pluripotent stem cells are induced pluripotent stem cells (IPS).

16. The method of preparing a retinal tissue unit according to one of claims 12 to 15, **characterized in that** it comprises a further step of loading a surgical implantation device suitable for injection into the eye with said tissue unit.

17. A kit for implanting tissue units according to one of claims 1 to 11 into the eye, **characterized in that** it comprises:
- between 1 and 10,000 tissue units according to one of claims 1 to 11,
- a surgical implantation device suitable for implanting said tissue unit(s) into a human eye.

18. The kit for implanting tissue units into the eye according to claim 17, **characterized in that** it comprises:
- between 1 and 10,000 tissue units according to claim 11,
- hydrogel capsule removal means,
- a surgical implantation device suitable for implanting said tissue unit(s) into a human eye.
